# EUROPEAN PATENT APPLICATION

(11) **EP 1 201 738 A1**
(43) Date of publication of application: **02.05.2002**
(21) Application number: 00203775.2
(22) Date of filing: 30.10.2000
(51) Int. Cl.: C11B 9/00, A61K 7/46, C11D 3/50

(54) **Fragrance composition comprising cyclohexadecanone**

(71) Applicant: PFW AROMA CHEMICALS B.V., 3771 ME Barneveld (NL)
(72) Inventor: Conboy, Frank Edward, 3852 EB Ermelo (NL); Lenselink, Willem, 3781 VC Voorthuizen (NL); van Manen, Anton Pieter Johan, 3881 CM Putten (NL)
(74) Representative: Prins, Adrianus Willem

(57) **Abstract**

The invention relates to a fragrance composition comprising a cyclic ketone. In particular such a composition provides a composition with improved substantivity, that can be used in various fragrance products. Furthermore the invention relates to employing such a composition in a range of processes, in which odour plays a role.

## Description

The invention relates to a fragrance composition comprising a cyclic ketone.

Cyclic ketones and structural analogues thereof are well known and much used ingredients of perfume compositions. Cyclic ketones are naturally occurring compounds, and have been obtained from both animals and plants such as musk deer, muskrats and civet cats as examples of animal origin and ambrette seeds or angelica as examples of vegetable origin. Macrocyclic ketones and derivatives thereof giving a more or less musky odour are also referred to as macrocyclic musks.

According to one of the standard works in fragrance chemistry by S. Arctander, one of the finest and most efficient fixative compound known in this family is cyclopentadecanone (*e.g.* Exaltone ®, by Firmenich), which has a strong musky odour [Perfume and Flavor Chemicals, S. Arctander (1969), Montclair, N.J.(USA)]. The other cyclic ketones of commercial importance are 3-methylcyclopentadecanone-1 *(e.g* Muscone®, by Firmenich), and cycloheptadecen-9-one-1 *(e.g.* Civettone ®, by Firmenich), [Braja D. Mookherjee in Fragrance Chemistry, the Science of the Sense of Smell, Ed. E.T. Theimer (1982) Academic press, New York]. Other known cyclic ketones are regarded as having inferior fragrance capabilities in comparison to the three compounds mentioned above. Some of them can be used as reagents for the synthesis of the already mentioned macrocyclic musks, such as cyclopentadecanone , which can be synthesised from cyclohexadecanone [Tetsuya Kato, Bull. Chem. Soc. Jpn (1980), 53(3), p. 823-4].

Apart from the fragrance being appreciated by a consumer, an important technical factor in perfume compositions is the substantivity of the composition to the substrate. Substantivity is an important factor in the persistence of *e.g.* a perfume on the skin, a perfume in shampoos or conditioners on the hair or a perfume in a detergent or fabric softener on textile fibres. Less substantive compositions would require a higher amount of fragrance compounds, in order to remain effective for an equal period, but this could lead to too high a concentration of fragrance compounds, resulting in a less pleasant odour.

Substantivity of a compound is a term used in relation to the degree of the attractive and repulsive forces between said compound and the solid support or substrate upon which it has been deposited. The degree of substantivity is affected by the way in which the perfume is delivered to the surface, *e.g.* in a washing/laundering process. This delivery can give rise to extra barriers. The degree of substantivity of a compound depends on both the capability of the compound to cross delivery barriers and how well the compound retains on a substrate after delivery. The latter effect, referred to as retention, is sometimes also used to indicate how well a perfume composition sticks to a substrate, but retention only relates to the behaviour of a perfume after it has successfully adhered to the substrate. In contrast to substantivity, retention does not include an indication of how well the delivery barriers are crossed by a fragrance compound or composition. Hence, a compound may - despite a good retention - be a poor fragrance compound in *e.g.* a personal care product, or a washing or laundering product if it does not manage to cross the barrier from product to substrate sufficiently, *i.e.* if it has too low a substantivity. Examples of applications in which a perfume composition have to take such barriers are the washing of laundry, hair, skin or surfaces. This involves the perfume transfer from an aqueous detergent solution or dispersion to a substrate, such as skin, hair, a textile, a ceramic or a surface. The nature of a substrate also influences substantivity; under comparable conditions of application, perfumes are in general a lot less substantive on nylon than on wool; cotton occupies an intermediate position. Substantivity can further be complicated by several factors, including temperature during washing and thereafter, pH value and the presence of other compounds.

When choosing an appropriate fragrance compound, another important factor to consider is the biodegradability of the perfume ingredients. Cyclic ketones used in fragrance compositions are generally known as having a good biodegradability, which is especially an advantage for use in shampoos, detergents and other products that often are discharged into the environment. Naturally the perfume ingredients should not invoke allergenic reactions, when used in a composition that may come into contact with animals or humans.

Particularly in commodity products such as detergents, the prices of the ingredients play an important role. For that reason the expensive cyclic ketones have not been applied to a great extent to other applications than fine perfumery in the past. Fine perfumery products are perfumes for direct application to the body or for use in cosmetic products. The use of some macrocyclic ketones in soap fragrances has not produced satisfactory results. This lack of positive results can probably at least partially be explained from the substantivity of these compounds being too low in soap applications. In perfumes for laundry treatment products, the substantivity of macrocyclic musks compared to other perfume ingredients is variable.

According to WO 98/32820 the substantivity of macrocyclic musks is reasonable for some, but most are not more than moderately substantive. Taking into account conditions *(e.g.* temperature, pH) in which *e.g.* detergents for washing laundry, shampoos or bath gels are used, a higher substantivity than obtainable from one of these macrocyclic musks, is usually necessary for a satisfactory use.

In WO 98/32820 several compositions are disclosed for treating skin, hair or textiles. These compositions comprise at least two of the macrocyclic musks hexadecanolide, cyclopentadecanone and pentadecanolide, which mixtures possess enhanced substantivity in comparison to their unsatisfactory substantivity when used separately.

Of the class of macrocyclic ketones, other compounds have never been regarded as useful fragrance compounds other than as compounds of academic interest or possibly as precursors for suitable fragrance compounds such as cyclopentadecanone, 3-methylcyclopentadecanone-1 or cycloheptadecen-9-one-1.

The present invention aims to provide a novel range of fragrance compositions with improved substantivity compared to fragrance compositions which have been employed until now in various applications. Thus the present invention may also provide extra possibilities for the use of various fragrance compounds of diverse chemical structures, in particular those of which the applicability in certain fragrance compositions has been troublesome due to their low substantivity until now, by employing such compounds in compositions according to the present invention.

Surprisingly it has been found that cyclohexadecanone has a significantly improved substantivity when compared to closely related commercial macrocyclic ketone fragrance chemicals such as cyclopentadecanone.

Cyclohexadecanone is found to be substantive enough in compositions according to the invention to be used in absence of another fragrance or fixating compound on various substrates such as skin, hair, wool, cotton, other textile fibres, ceramics, leather or coated surfaces. Moreover from compositions according to this invention comprising cyclohexadecanone , an appreciated odour can be experienced, despite the general expectations. The invention accordingly relates to a fragrance composition comprising cyclohexadecanone.

It is an option to use cyclohexadecanone according to the present invention instead of more expensive fragrance compounds of lesser substantivity. Nonetheless, within the scope of the present invention it is possible to use cyclohexadecanone in combination with one or more other fragrance compounds *e.g.* to allow use of such other fragrance compound or compounds in lesser concentrations than is usual until now. Furthermore it is commonly known in the field of perfume chemistry that often a desired scent is achieved after delicately mixing a variety of different fragrance compounds. Such an embodiment of the present invention could *e.g.* be a fragrance composition comprising cyclohexadecanone and further comprising commonly used fragrance compounds and/or fragrance compounds that would not be substantive to a satisfactory degree when applied without a more substantive compound such as cyclohexadecanone. Thus, according to the present invention, cyclohexadecanone can also be used to alter, enhance and/or reinforce the aroma characteristics of one or more other natural and/or synthetic compounds. According to the present invention, cyclohexadecanone can further be used for enhancing the substantivity of a perfume composition.

Examples of additional fragrance compounds that may be used in a composition according to the invention include certain natural extracts of vegetable or animal origin; natural oils; synthetic oils; alcohols, aldehydes, ketones, esters, lactones, ethers, hydrocarbons, nitriles, and other classes of chemical compounds that can be used alter or enhance the scent of other compounds, compositions or the environment. Such ingredients are mentioned, for example, in S. Arctander, Perfume and Flavor Materials of Natural Origin (Elizabeth, N. J. 1960), S. Arctander, Perfume and Flavor Chemicals (Montclair N.J. 1969) and in Flavor and Fragrance Materials, 1997, Allured Publishing co. Wheaton Ill. USA or earlier versions of this periodical.

A composition according to the present invention may have several advantages, such as allowing the use of a composition comprising a lower concentration of a fragrance compound or its use in a simpler composition without adversely affecting the persistence of a fragrance composition on a substrate. Furthermore, cyclohexadecanone is considerably less expensive than cyclopentadecanone and some other commonly used compounds.

Cyclohexadecanone may either be available from a natural source or can be prepared by a method known in the art, *e.g.* via intramolecular ring closure according to Ruzicka in which a desired cyclic ketone is derived from a dicarboxylic acid [Braja D. Mookherjee, Fragrance Chemistry, the Science of the Sense of Smell, Ch. 12, Ed. E.T. Theimer (1982) Academic press, New York].

In standard literature, such as in "Perfume and Flavor Chemicals" [(1969), Montclair, N.J.(USA)] by S. Arctander, cyclohexadecanone is reported as a compound that does not offer sufficiently interesting notes, power or effect that it could compete with the variety of efficient fragrances currently available.

Nonetheless, according to the present invention, cyclohexadecanone has been found to give rise to an excellent substantivity to fragrance compositions, even in applications where environment conditions such as pH, temperature, electrolyte concentration or substrate are known to have a detrimental effect on substantivity of many conventional fragrance compositions. Typical examples of such applications are laundering processes, hair treatment, skin treatment, fabric treatment, dish washing, household cleaning, eau de cologne, perfume or deodorant applications, and so on.

The improved substantivity of cyclohexadecanone for use in a composition according to the invention compared to the related macrocyclic musk compounds allows for example the use of cyclohexadecanone in a smaller amount than the commonly used compounds in such a perfume composition or to use a composition according to the invention in a process, particularly a process demanding a high substantivity of the perfume composition in order to be effective or efficient, *e.g.* in a laundering process, fabric softening process, ironing process, bleaching process, washing process or use in shampoo, bath gel, bath salt, bath soap and the like, in a personal care product, alcoholic fine fragrance or in another composition according to this invention.

The invention accordingly provides fragrance compositions and products-comprising cyclohexadecanone - for treating substrates, including skin, textiles, hair, leather, carpets, cars, ceramic or other surfaces. Thus the invention also encompasses the use of cyclohexadecanone in various products, which may also comprise other, conventional, accompanying ingredients, of which the skilled professional will know how to employ them. Typical examples of such products according to the invention are detergents, such as detergent granules, detergent tablets and liquid detergents, soaps, fabric treatment products such as fabric softeners, fabric care products, fabric sprays, fabric deodorants, ironing aiding products, dryer added product, optical whiteners, odour masking products, personal care products, deodorant products, antiperspirants, air fresheners, perfumes, colognes, after shave lotions, bath oils, bath salts, lacquers, brilliantines, pomades, shampoos, creams, deodorants, hand lotions, sun screens, talcs, dusting powders, face powders, masking agents, bleach products, cleaners, dish washing products, scourers, toilet cleaners, carpet cleaners, paints, inks, shoe polish, automobile wax, cosmetics, functional products, household products, soaps, cleaning products, toilet cleaners, candles, lamp oils, incense products, air fresheners, talcs, diapers, panty liners, absorbents, bleach products, alcoholic perfumes and air fresheners. Preferred products according to the invention include detergents, shampoos and personal care products.

A Composition according to the invention can be used in combination with various additives such as surfactants, carriers, solvents, dispersants, emulsifiers, , aerosols propellants, odour release influencing agents, vehicles, other fragrance compounds, and the like.

It has been found that as little as 0.05% by weight of cyclohexadecanone can be used to alter the odour effect of a fragrance composition. The quantity in which cyclohexadecanone is used in a perfumed composition, material may vary within wide limits depending upon several factors including, the desired odour, the characteristics of other ingredients, the nature of the end-product, the nature of the substrate, the concentration of electrolytes and other compounds, the temperature and/or the pH when using the composition in a process such as hair or skin treatment, laundry washing, dish washing, etc., but also considerations of a financial nature.

In perfume compositions an amount of 0.05% by weight or more of cyclohexadecanone will generally have an odorous effect. This effect itself can be described as a warm floral woody diffusive musk odour with a sparkling ozonic top and a sweet musk dry-out. Preferably the relative amount of cyclohexadecanone in a composition is between 0.1% by weight and 50% by weight. More preferably the relative amount in a perfume composition according to the invention is between 0.1% by weight and 30% by weight. In some products, for example in detergent granules, the amount is preferably between 0.1% by weight and 20% by weight. It has been found that within this concentration range of cyclohexadecanone, the substantivity has its optimum for most applications, and that the odour characteristics are in general very well appreciated.

Compositions according to the invention may have any physical form, *e.g.* a granular form, powder, emulsion, paste, liquid, aerosol, gel of a solid shape.

In a preferred embodiment, a composition according to the invention is a detergent composition, comprising cyclohexadecanone and one ore more surfactants, preferably selected from the classes of anionic surfactants, such as carboxylates, acylated protein hydrolysates, sulfonates, sulphates including alkylbenzenesulfonates, alkylarenesulfonates, lignosulfates, naphtalene-sulfonates, -olefinsulfonates and sulfonates with ester, amide or ether linkages, sulfated products such as sulfated alcohols, sulfated alcohol ethoxylates, sulfated alkylphenols, sulfated ethoxylated alkylphenols, sulfated acids, sulfated amides and sulfated esters, sulfated natural oils and fats, mono- and diesters of phosphate or alkyl phosphate esters an their salts; cationic surfactans such as primary, secondary, tertiary or quaternary ammonium salts, *e.g.* derived from fatty acids or rosin acids, from oxygen containing amines such as amine oxides, ethoxylated amines, imidazolines and alkoxylates of ethylenediamine and the like; amphoteric surfactants such as imidazolinium derivatives; or non-ionic surfactants such as ethoxylates, propoxylates, carboxylic esters such as glycerol esters, polyoxyethylene esters, anhydrosorbitol esters, ethoxylated anhydrosorbitol esters, natural fats, oils, waxes, ethoxylated and glycol esters of fatty acids, and polyalkylene oxide block copolymers, *e.g.* derived from propylene, butylene, styrene and cyclohexene oxides. The concentration of a surfactant in an embodiment according to the invention is not particularly limited. The preferred surfactant concentration will depend upon the application and the type of surfactant. In an embodiment, *e.g.* a particular bleach product the concentration may be preferably lower than 1 wt. %. whereas in another embodiment, *e.g.* a soap or a washing powder the concentration may be higher than 99 %. A composition according to the invention comprising a detergent has been found to be a particularly suitable example of a composition for cleaning purposes wherein cyclohexadecanone demonstrates a particularly satisfactory substantivity and a desirable odour pattern.

A more preferred embodiment further comprises a carrier, such as a polysaccharide, *e.g.* a cellulose, an alginate, a pectin, an ester of a polysaccharide, another derivative of a polysaccharide or a combination thereof. The preferred carrier concentration will depend upon the application, other compounds in the composition and the type of carrier. If a carrier is present, the carrier concentration will typically be in the range of 10 to 50% by weight. These carriers proved to be particularly suitable compounds to further enhance a desired fragrance effect of compositions according to the invention.

A preferred embodiment of the present invention is a detergent composition for laundry washing - *e.g.* prepared according to a conventional method-comprising cyclohexadecanone, admixed with a surfactant, such as mentioned above and one or more other conventional ingredients for detergent compositions. Such a composition may have any physical form, *e.g.* a granular form, powder, liquid, gel of a solid shape.

In a preferred embodiment a detergent composition according to the invention may be used in a laundering process, in which said composition is mixed with a solvent - usually water - and the laundry. The laundering process may further comprise one or more of the following steps in any suitable order, each applied as often as preferred: soaking in water and detergent, during which the laundry may be continuously or intermittently in motion; rinsing with water, possibly the addition of extra detergent, fabric softener and the like; optional additional rinsing, centrifugation; drying at ambient or elevated temperature and optionally ironing. The function of the detergent is to remove dirt from the laundry, whereas cyclohexadecanone and optionally one or more other fragrance compounds are transferring from the detergent composition to the laundry to which said compound or compounds should adhere throughout the process and a significant time - preferably several days - thereafter, to give a desired scent for a reasonable period.

Typical laundering temperatures within an embodiment according to the invention are from ambient up to 95°C, in a more preferred embodiment in a temperature range from 40 to 60°C. Furthermore, especially in laundering processes, pH may play an important role. A preferred detergent composition according to the invention can be employed in the range of pH 3 to pH 12, more preferably at a pH in the range of 4 to 11 wherein pH should be interpreted as the pH value of a composition or product according to the invention in water at 25°C. In case an embodiment of the invention comprises the use of a composition according to the invention in an environment leading to an altered solvent autoprotolytic constant (pK_{w}), the preferred pH range should be interpreted based upon the pH range at 25°C in water. *E.g.* at a temperature of 50°C the pK_{w} of a solvent is 13.3 instead of 14 (of water at 25°C), accordingly the neutral pH is has changed from 7 (25°C) to 6.65.

Another preferred embodiment of the present invention is a shampoo composition - *e.g.* prepared according to a conventional method- comprising cyclohexadecanone, admixed with a surfactant, such as mentioned above and one or more other conventional ingredients. Such a composition may have any suitable form, *e.g.* a liquid or a gel. A more preferred embodiment further comprises a carrier, such as a polysaccharide.

In a preferred embodiment a shampoo composition according to the invention may be used in a process to wash human or animal hair, in which said shampoo composition is lathered into the hair. The detergent has the function to remove soil such as bodily fats, dust, etc., from the hair, whereas cyclohexadecanone and optionally one or more other fragrance compounds are transferring from the shampoo composition to the hair. After a certain period, typically of up to one or a few minutes, but possibly longer, the detergent, soil and excess of the other shampoo compounds are removed from the hair, by rinsing it with water of typically ambient temperature or up to 60°C, preferably of a temperature up to 50°C. It is stressed that water of a sub-ambient temperature can be used satisfactorily well within the scope of the invention. The pH will normally be in a relatively small range around the neutral pH, in order to avoid irritation on the skin. During rinsing cyclohexadecanone and -optionally - other fragrance compounds, should adhere to the hair and a significant time thereafter, to give a desired scent for a reasonable period.

In a personal care product, a composition according to the present invention comprises cyclohexadecanone, and preferably a surfactant *e.g.* such as mentioned above, and a carrier, *e.g.* a polysaccharide.

Furthermore a product, such as a personal care product, may comprise one or more emulsifiers, *e.g.* anionic emulsifiers such as carboxylates, acylated protein hydrolysates, sulfonates, sulphates including alkylbenzenesulfonates, alkylarenesulfonates, lignosulfates, naphtalenesulfonates, -olefinsulfonates and sulfonates with ester, amide or ether linkages, sulfated products such as sulfated alcohols, sulfated alcohol ethoxylates, sulfated alkylphenols, sulfated ethoxylated alkylphenols, sulfated acids, sulfated amides and sulfated esters, sulfated natural oils and fats, mono- and diesters of phosphate or alkyl phosphate esters an their salts; cationic emulsifiers such as primary, secondary, tertiary or quaternary ammonium salts, *e.g.* derived from fatty acids or rosin acids, from oxygen containing amines such as amine oxides, ethoxylated amines, imidazolines and alkoxylates of ethylenediamine and the like; amphoteric emulsifiers such as imidazolinium derivatives; non-ionic emulsifiers such as ethoxylates, propoxylates, carboxylic esters such as glycerol esters, polyoxyethylene esters, anhydrosorbitol esters, ethoxylated anhydrosorbitol esters, natural fats, oils, waxes, ethoxylated and glycol esters of fatty acids, and polyalkylene oxide block copolymers, *e.g.* derived from propylene, butylene, styrene and cyclohexene oxides. Furthermore a composition according to the invention may comprise other components that are typically used in personal care products. Like other compositions according to the invention a personal care product according to the invention may have any physical form, *e.g.* a granular form, powder, emulsion, paste, liquid, aerosol, gel of a solid shape.

In a preferred embodiment a personal care product according to the invention may be used in a process to treat skin, in which said personal care product is lathered onto the skin. The function of the detergent is to remove soil such as bodily fats, dust, cosmetics etc., from the skin, whereas cyclohexadecanone and optionally one or more other fragrance compounds are transferring from the personal care product to the skin. After a certain period, perhaps less than a minute, but possibly up to a few minutes or longer, the detergent, soil and excess of the other personal care product compounds are removed from the skin, by rinsing it with water of, typically ambient temperature or up to 60°C, preferably up to 50°C. It is stressed that water of sub-ambient temperature can be used satisfactorily well within the scope of the invention. During rinsing, cyclohexadecanone - and optionally other fragrance compounds-should adhere to the skin and a significant time thereafter, to give a desired scent for a reasonable period.

In yet another preferred embodiment the composition according to the invention is a perfume composition comprising a mixture of fragrances, including cyclohexadecanone, wherein said composition is typically in a solvent such as water and/or one or more alkanols or a mixture thereof. More preferably said composition further comprises a carrier compound selected from the already mentioned carrier compounds.

Substantivity of cyclohexadecanone was evaluated through smelling by a perfumery odour testing panel skilled in the art, according to standard procedures known in the art [The Use of Fragrance in Consumer Products, J.S. Jellinek (1975), John Wiliey & Sons, NY (USA)], after treating samples of a substrate with a perfumed product, such as an acid cleaner, cream rinse, toilet cleaner, deo stick, fabric softener, deo pump spray, eau de toilette, hand cream, liquid detergent, shampoo, talc, bar soap, all purpose cleaner, detergent powder, compact detergent powder, liquid bleach , said perfumed product comprising cyclohexadecanone or another fragrance compound for comparison respectively.

The invention is further illustrated in the following examples:

### EXAMPLE 1

In a well-stirred one-litre glass autoclave , a mixture of 60.3 g of 8-cyclohexadecen-1-one (Animusk®, Fragrance Resources) 450 ml 96% ethanol and 1 g palladium 10 % on carbonwas hydrogenated at room temperature in a 5 bar hydrogen atmosphere during 1.5 hours Thereafter the mixture was filtered through a filter paper on a Büchner funnel and subjected to high vacuum fractionation through a Spaltrohr® column (Fischer) yielding 45.9 g of cyclohexadecanone boiling point 75°C at 0.01 mbar, melting point 67.7°C, purity 99.4% (determined by GC).

### EXAMPLE 2

Two batches of non-perfumed Compact detergent powder (Ariel Futur®, Procter & Gamble) were perfumed on a 0.8 wt.% level with respectively a 10 wt.% cyclopentadecanone solution in diethyl phtalate (as a reference to the state of the art) or a 10 wt.% cyclohexadecanone solution in diethyl phtalate. Standard 40°C main wash programs (50 g detergent powder, 600 g laundry, 1 hr wash cycle with 9 l. water, four rinse cycles with respectively 12, 20, 12 and 20 l. water, centrifugation cycle at 800 rpm) were performed with several batches of household cotton towel, cotton terry cloth and cotton/synthetic cloths in a standard household washing machine (Philips Whirlpool, AWG 744), wherein one half of the batches were washed with a perfumed detergent according to the invention and the other half of the batches were washed with the perfumed detergent powders comprising cyclopentadecanone.

After washing the odour was evaluated in the head space of the drum of the washing machine directly after the washing cycle, and the odour of the laundry was evaluated in the wet and air-dried stages by a perfumers panel. The odour of the drum head space for the washing cycle with cyclohexadecanone was to be found clearly recognisable, qualified as more fresh, good radiant ozonic, musk and significantly stronger in comparison to the reference batch comprising cyclopentadecanone. The odours of all wet cloths washed with the detergent comprising cyclohexadecanone, were found to be clearly recognisable, qualified as floral, diffusive, fresh musk and significantly stronger in comparison to the reference cyclopentadecanone. The odour of all 12 hours line-dried cloths washed with a detergent comprising cyclohexadecanone were found to be clearly recognisable, qualified as fresh, woody, sparkling musk and significantly stronger compared to the reference cyclopentadecanone.

### EXAMPLE 3

A test similar to Example 2 was performed, now with cyclohexadecanone on a 0.4% level and instead of cyclopentadecanone, Lilial was used on a 0.4% level as the reference. Lilial is generally accepted in the art as a reference for substantivity testing, since Lilial has a good substantivity. Furthermore Lilial is clearly recognisable in the odour of the head space of the drum of the washing machine and on all wet and dried cloths.

The odour of the drum head space used for the washing cycle with cyclohexadecanone was found to be strong recognisable, qualified as significantly stronger compared to the reference test with Lilial.

The odour from all wet cloths as well as all dried clothes washed with the detergent perfumed with cyclohexadecanone were found to be strong recognisable, qualified as much stronger in comparison to the reference test with Lilial.

### EXAMPLE 4

Perfume compositions, especially suited for use in laundry detergents were prepared by mixing the following ingredients:

| Component (Supplier) | Composition A (reference) | Composition B |
|---|---|---|
| | (parts by weight) | (parts by weight) |
| Rosenitrile (PFW) | 10 | 10 |
| Lilial (GIV) | 20 | 20 |
| Cyclomyral (PFW) | 20 | 20 |
| Sandalore (GIV) | 20 | 20 |
| Tilianol Super (PFW) | 60 | 60 |
| a-Methylcinnamic alcohol | 80 | 80 |
| Dihydro Isojasmonate (PFW) | 80 | 80 |
| Hedione (FIRM) | 80 | 80 |
| Citronellol 700 (BBA) | 150 | 150 |
| Orange Isolate (PFW) | 400 | 400 |
| Dipropylene glycol | 80 | - |
| Cyclohexadecanone (according to Example 1) | - | 80 |
| Total | 1000 | 1000 |

Wash tests similar to Example 2 were performed with Compact detergents that were respectively perfumed at a 0.4% level with composition A (as the reference example) or with perfume composition B (comprising cyclohexadecanone). Composition B showed a surprisingly better substantivity on both wet and dried laundry and gave rise to a more fresh floral and diffusive musk odour effect.

### EXAMPLE 5

Rose perfume compositions, especially suited for use in cosmetic applications, were prepared by mixing the following ingredients:

| Component (Supplier) | Composition A (reference) | Composition B |
|---|---|---|
| | (parts by weight) | (parts by weight) |
| Methyl Octalactone (PFW) | 1 | 1 |
| Oxambrane (PFW) | 1 | 1 |
| Leaf Acetal (IFF) | 1 | 1 |
| Galbex (FIRM) | 2 | 2 |
| Heliotropin | 10 | 10 |
| Hypo Lem (IFF) | 15 | 15 |
| Rosenitrile (PFW) | 20 | 20 |
| Sandalore (GIV) | 20 | 20 |
| Dihydromyrcenol | 30 | 30 |
| Phenoxyethyl isobutyrate | 30 | 30 |
| Lavandin oil Abrialis (Bontoux) | 30 | 30 |
| Cyclohexyl salicilate | 30 | 30 |
| Cynthaflor (PFW) | 40 | 40 |
| Cyclomyral (PFW) | 40 | 40 |
| Tilianol Super (PFW) | 40 | 40 |
| p-t-Butylcyclohexyl acetate | 40 | 40 |
| Lilial (GIV) | 50 | 50 |
| Tetrahydrolinalool | 70 | 70 |
| Isononyl acetaat | 70 | 70 |
| Dihydro Isojasmonate (PFW) | 70 | 70 |
| Lemon isolate (PFW) | 70 | 70 |
| Citronellol 700 (BBA | 250 | 250 |
| Dipropylene glycol | 80 | - |
| Cyclohexadecanone (According to Example 1) | - | 80 |
| Total | 1000 | 1000 |

Perfume B showed a significantly better substantivity to the skin and a more sparkling floral diffusive odour effect was experienced.

## Claims

1. A fragrance composition comprising cyclohexadecanone.

2. A composition according to claim 1, wherein said composition comprises a surfactant.

3. A composition according to claim 1 or 2, wherein said composition comprises a carrier.

4. A composition according to any of the claims 1-3, wherein said composition comprises a solvent.

5. A composition according to any of the preceding claims, wherein said composition comprises from 0.1 to 50 wt.% of cyclohexadecanone.

6. A product comprising a composition according to any of the preceding, wherein said product is selected from the group of detergents, soaps, fabric softeners, fabric care products, fabric sprays, fabric deodorants, ironing aiding products, dryer added product, optical whiteners, odour masking products, personal care products, air fresheners, perfumes, colognes, after shave lotions, bath oils, bath salts, lacquers, brilliantines, pomades, shampoos, creams, deodorants, antiperspirants, hand lotions, sun screens, talcs, dusting powders, face powders, masking agents, bleach products, cleaners, dish washing products, scourers, toilet cleaners, carpet cleaners, paints, inks, shoe polish, candles, lamp oils, incense products, diapers, panty liners, absorbents and automobile wax.

7. A product according to claim 6, chosen from the group of detergents, shampoos and personal care products.

8. Use of cyclohexadecanone, to alter, enhance and/or reinforce the aroma characteristics of another natural or synthetic compound.

9. Use of cyclohexadecanone for enhancing the substantivity of a perfume composition.
